# EUROPEAN PATENT APPLICATION

(11) **EP 4 353 900 A1**
(43) Date of publication of application: **17.04.2024**
(21) Application number: 22201071.2
(22) Date of filing: 12.10.2022
(51) Int. Cl.: D06M 15/285

(54) **FACIAL MASK EXHIBITING HIGH THERMAL CONDUCTIVITY**

(71) Applicant: BIC Violex Single Member S.A., 145 69 Anoixi (GR)
(72) Inventor: Antonakis, Ion - Ioannis, 145 69 Anoixi (GR)
(74) Representative: Peterreins Schley

(57) **Abstract**

In a first aspect, the present disclosure relates to a facial mask comprising a carrier material and a thermoresponsive material configured to release water above a threshold temperature and absorb water below a threshold temperature, wherein the facial mask exhibits a thermal conductivity of at least 0.1 W/mK at 20°C, when the facial mask is saturated with water.

## Description

### Technical Field

The present disclosure relates to the field of facial masks. More specifically, the present disclosure relates to facial masks exhibiting a high thermal conductivity.

### Background

Facial masks are masks applied to the face for skin care applications. The facial mask may comprise skin care agents, such as minerals vitamins and/or fruit extracts. Facial masks may be in the form of a creamy or thick paste. The paste may then be applied by the user to his face, usually leaving blank the mouth, nostrils and eyes.

Alternatively, facial masks may be in the form of a pre-manufactured sheet, also referred to as sheet masks. The pre-manufactured sheets may already comprise opening for the mouth, eyes and nostrils and may therefore be simply applied by the user by placing the mask on the face.

Women are still the primary users of skin care products including facial masks. However, the interest and market share of men in skin care products has increased. A common skin care product used by men is aftershave, which is a fluid applied to the skin after shaving. Aftershave is commonly applied to prevent razor burn, for which it may comprise for example astringents. However, aftershave is by its nature a non-reusable product.

The present disclosure relates to improved products for facial skin care.

### Summary

In a first aspect, the present disclosure relates to a facial mask comprising a carrier material and a thermoresponsive material configured to release water above a threshold temperature and absorb water below a threshold temperature, wherein the facial mask exhibits a thermal conductivity of at least 0.1 W/mK at 20°C, when the facial mask is saturated with water.

In some embodiments, the threshold temperature may be between about 30°C to about 45°C, more specifically between about 33°C to about 40°C, and in particular between about 35°C to about 38°C.

In some embodiments, the facial mask may exhibit a thermal conductivity of at least 0.2 W/mK, more specifically at least 1 W/mK and in particular at least 5 W/mK at 20°C, when the facial mask is saturated with water.

In some embodiments, the carrier material may exhibit a thermal conductivity of at least 0.2 W/mK, more specifically at least 1 W/mK and in particular at least 5 W/mK at 20°C.

In some embodiments, the carrier material may be flexible.

In some embodiments, the facial mask may comprise between about 10 wt.-% to about 80 wt.-%, more specifically between about 20 wt.-% to about 70 wt.-% and in particular between about 30 wt.-% to about 60 wt.-% of the carrier material, relative to the total weight of the facial mask excluding water.

In some embodiments, the facial mask may comprise between about 10 wt.-% to about 80 wt.-%, more specifically between about 20 wt.-% to about 70 wt.-% and in particular between about 30 wt.-% to about 60 wt.-% of the thermoresponsive material, relative to the total weight of the facial mask excluding water.

In some embodiments, the carrier material may exhibit a Young's modulus between about 0.1 GPa to about 60 GPa, more specifically 0.5 GPa to about 10 GPa and in particular, between about 1 GPa to about 5 GPa.

In some embodiments, the carrier material may comprise a polymer.

In some embodiments, the carrier material may comprise fibers, in particular polymer fibers.

In some embodiments, the carrier material may be a woven material, in particular the carrier material may be a woven fabric.

In some embodiments, the thermoresponsive material may be embedded within the carrier material.

In some embodiments, the fibers may have a diameter between about 100 µm to about 2000 µm, more specifically between about 300 µm to about 1000 µm.

In some embodiments, the carrier material may comprise fillers configured to increase the carrier material's thermal conductivity, more specifically inorganic fillers and in particular a metal, graphite, carbon nanotubes and/or a ceramic.

In some embodiments, the filler may be in the form of particles, sheets or fibers, in particular sheets or fibers.

In some embodiments, the metal may comprise copper particles and/or copper fibers.

In some embodiments, the ceramic may comprise boron-nitride.

In some embodiments, the carrier material may comprise between about 0.1 wt.-% to about 70 wt.-%, more specifically between about 0.5 wt.-% to about 60 wt.-% and in particular between about 1.5 wt.-% to about 50 wt.-% of the inorganic filler, relative to the total weight of the carrier material including the filler.

In some embodiments, the carrier material may comprise ultrahigh molecular weight polyethylene.

In some embodiments, the thermoresponsive material may comprise a thermoresponsive polymer.

In some embodiments, the thermoresponsive material may comprise a hydrogel.

In some embodiments, the thermoresponsive material may comprise an interpenetrating polymer network, more specifically an interpenetrating polymer network comprising the thermoresponsive polymer and the hydrogel.

In some embodiments, the thermoresponsive polymer may undergo a conformational change at a lower critical solution temperature, in particular the thermoresponsive polymer may be configured to be in an expanded state below the lower critical solution temperature and a collapsed state above the lower critical solution temperature.

In some embodiments, the lower critical solution temperature of the thermoresponsive material may be between about 30°C to about 45°C, more specifically between about 33°C to about 40°C, and in particular between about 35°C to about 38°C.

In some embodiments, the threshold temperature may be the lower critical solution temperature.

In some embodiments, the thermoresponsive polymer may comprise poly(N-isopropylacrylamide) and/or poly(N-vinylcaprolactam).

In some embodiments, the hydrogel may comprise a network of crosslinked polymer chains, wherein the crosslinked polymer chains comprise hydrophilic polymer chains.

In some embodiments, the hydrogel may comprise sodium alginate, agarose, methylcellulose, hyaluronan, collagen, gelatin, fibrin, elastin like polypeptides, polyvinyl alcohol, polyethylene glycol, sodium polyacrylate, acrylate polymers, copolymers thereof and/or mixtures thereof.

In some embodiments, the hydrogel may comprise a polyacrylamide-carbon nanotube-calcium chloride hydrogel.

In some embodiments, the specific water absorption capacity of the thermoresponsive material may be at least about 0.1 g/g, more specifically at least about 1.2 g/g, and in particular at least about 9.0 g/g.

In some embodiments, the average molar mass of the thermoresponsive polymer may be between 1000 g/mol to about 100000 g/mol, more specifically between about 5000 g/mol to about 70000 g/mol and in particular between about 10000 g/mol to about 50000 g/mol.

In some embodiments, the thermoresponsive material may comprise between about 10 wt.-% to about 80 wt.-%, more specifically between about 20 wt.-% to about 70 wt.-% and in particular between about 30 wt.-% to about 60 wt.-% of the thermoresponsive polymer, relative to the total weight of the thermoresponsive material excluding water.

In some embodiments, the thermoresponsive material may comprise a salt, in particular potassium chloride.

In some embodiments, the thermoresponsive polymer may be more hydrophilic at temperatures below the lower critical solution temperature than at temperatures above the lower critical solution temperature.

In some embodiments, the thermoresponsive polymer may be more hydrophobic at temperatures above the lower critical solution temperature than at temperatures below the lower critical solution temperature.

In some embodiments, the facial mask may comprise a bactericidal agent, in particular the thermoresponsive material may comprise the bactericidal agent.

In some embodiments, the facial mask may comprise skin care ingredients, in particular water-soluble skin care ingredients.

In some embodiments, the facial mask may comprise a cooling agent.

In some embodiments, the cooling agent may be configured to trigger cold-sensitive receptors, in particular the cold-sensitive TRPM-8 receptors.

In some embodiments, the cooling agent may be an essential oil, in particular menthol.

In a second aspect, the present disclosure relates to a method for producing a facial mask, wherein the method may comprise embedding a material configured to release water above a threshold temperature and absorb water below a threshold temperature into a carrier material exhibiting a thermal conductivity of at least 0.1 W/mK.

### Brief Description of the Drawings

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different Figures indicate like or similar features.

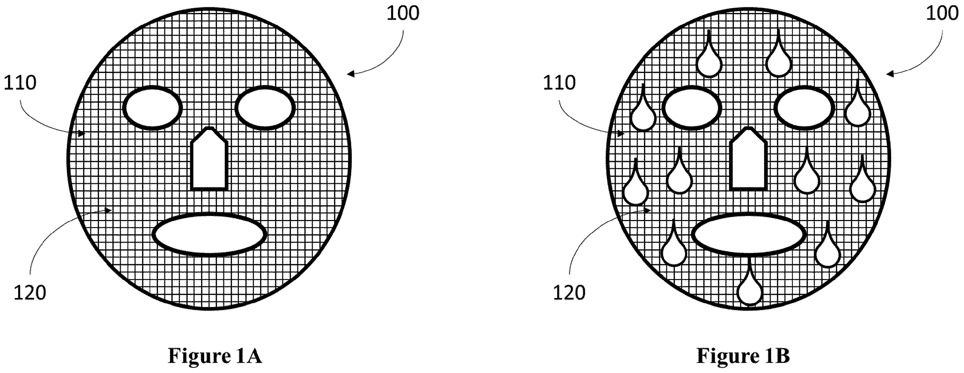

**Figure 1A** shows a facial mask according to the first aspect at a temperature below a threshold temperature.
**Figure 1B** shows a facial mask according to the first aspect at a temperature above a threshold temperature

### Detailed Description

Hereinafter, a detailed description will be given of the present disclosure. The terms or words used in the description and the aspects of the present disclosure are not to be construed limitedly as only having common-language or dictionary meanings and should, unless specifically defined otherwise in the following description, be interpreted as having their ordinary technical meaning as established in the relevant technical field. The detailed description will refer to specific embodiments to better illustrate the present disclosure, however, it should be understood that the presented disclosure is not limited to these specific embodiments.

Shaving close to the skin, for example shaving with a straight razor or safety razor, may result in slight skin damage. In particular, shaving close to the skin may result in the removal of outer skin layers, which may in turn result in increased transepidermal water loss. Additionally, shaving close to the skin may result in small nicks and cuts in the skin, which may become inflamed. To reduce the risk of inflammation it may be useful to cool the skin.

Accordingly, in a first aspect, the present disclosure relates to a facial mask 100 comprising a carrier material 110 and a thermoresponsive material 120 configured to release water above a threshold temperature and absorb water below a threshold temperature, wherein the facial mask 100 exhibits a thermal conductivity of at least 0.1 W/mK at 20°C, when the facial mask 100 is saturated with water.

**Figure 1A** shows a schematic facial mask 100 according to the first aspect at a temperature below the threshold temperature, wherein the mask comprises a carrier material 110 and a thermoresponsive material 120.

The user may employ the facial mask 100 by first placing the facial mask 100 in water with a temperature below the threshold temperature, such that the temperature of the facial mask 100 remains or is brought below the threshold temperature. Thereby, the facial mask 100, more specifically the thermoresponsive material 120 comprised therein, may absorb water. The user may perform this "soaking action" prior to shaving or directly thereafter. Subsequently, the user can apply the mask to the face, whereby the temperature of the mask may be increased above the threshold temperature due to heat from the face, resulting in the release of the previously absorbed water, as schematically depicted in **Figure 1B****.** Due to the high thermal conductivity of the facial mask 100, the release of water may occur relatively quickly. The released water may replenish the water lost due to transepidermal water loss and/or prevent further water loss from the skin. Additionally, the released water may aid in cooling the skin. Moreover, the combination of the high thermal conductivity of the facial mask 100 and the thermoresponsive material 120 may reduce the time for the thermoresponsive material's 120 temperature being increased above the threshold temperature. Furthermore, the combination of the high thermal conductivity of the facial mask 100 and the thermoresponsive material 120 may increase the cooling rate. Water may evaporate, thereby removing the enthalpy of evaporation from the mask. The rate of water evaporation and thereby cooling may be increased by improving the transport of thermal energy from the skin's surface to the water, thus by increasing the thermal conductivity of the facial mask 100.

In some embodiments, the thermoresponsive material 120 may be embedded within the carrier material 110, as schematically shown in **Figure 1A** and **Figure 1B****.** Embedding the thermoresponsive material 120 into the carrier material 110 may improve the thermal conductivity between the carrier material 110 and the thermoresponsive material 120. Further, embedding the thermoresponsive material 120 into the carrier material 110, may furthermore protect the thermoresponsive material 120 from physical damage, in particular if the hardness of the carrier material 110 is higher than that of the thermoresponsive material 120 when it has absorbed water.

In some embodiments, the threshold temperature may be between about 30°C to about 45°C, more specifically between about 33°C to about 40°C, and in particular between about 35°C to about 38°C. The threshold temperature set to these ranges may configure the thermoresponsive material 120 to release water when placed on a user's face, as the user's body temperature may heat up the facial mask 100 to a temperature above the threshold temperature.

In some embodiments, the facial mask 100 may exhibit a thermal conductivity of at least 0.2 W/mK, more specifically at least 1 W/mK and in particular at least 5 W/mK at 20°C, when the facial mask 100 is saturated with water. The measurement of the thermal conductivity may be performed according to ISO 22007-2:2022. For example, the thermal conductivity may be measured using a "Trident" manufactured by the company C-Therm and a Flex-TPS Sensor.

The facial mask's 100 high thermal conductivity may be based on the carrier material 110, in particular the carrier material 110 may be exhibiting a high thermal conductivity. Hence, the carrier material 110 may exhibit a thermal conductivity of at least 0.2 W/mK, more specifically at least 1 W/mK and in particular at least 5 W/mK at 20°C.

The carrier material 110 may comprise a polymer. Furthermore, the carrier material 110 may comprise fibers, in particular polymer fibers. The fibers, in particular the polymer fibers may be woven. Hence, the carrier material 110 may be a woven material, in particular the carrier material 110 may be a woven fabric. The woven material may exhibit an increased tear resistance compared to individual fibers. Additionally, the woven material may exhibit an improved temperature distribution as the fibers are in thermal contact with one another.

In some embodiments, the fibers may have a diameter between about 100 µm to about 2000 µm, more specifically between about 300 µm to about 1000 µm. The aforementioned diameter ranges may provide the fibers with sufficient flexibility and tear resistance, in particular if woven into a fabric.

Polymers, in particular polymer fibers, may exhibit a higher flexibility compared to other materials. Hence, the carrier material 110 and/or facial mask 100 may be flexible. The flexibility of the carrier material 110 may result in the facial mask 100 being flexible. Due to this flexibility, the carrier material 110 and/or the facial mask 100 may better align with the user's face. The improved alignment may result in an improved replenishment of water to the skin and an improved thermal transfer between the skin and the facial mask 100, which may result in improved cooling of the skin. In some embodiments, the carrier material 110 may exhibit a Young's modulus between about 0.1 GPa to about 60 GPa, more specifically 0.5 GPa to about 10 GPa and in particular, between about 1 GPa to about 5 GPa.

The thermal conductivity of the facial mask 100, more specifically of the carrier material 110, may be increased using fillers. Hence, in some embodiments, the carrier material 110, in particular the polymer, may comprise fillers configured to increase the carrier material's 110 thermal conductivity, more specifically inorganic fillers and in particular a metal, graphite, carbon nanotubes and/or a ceramic. For example, the filler may be in the form of particles, sheets or fibers, in particular sheets or fibers. Fillers in the form of sheets or fibers may increase the thermal conductivity more efficiently, as these are more likely to form thermal contacts. The metal may comprise copper particles and/or copper fibers. Alternatively or additionally, the metal may comprise silver particles and/or silver fibers. The ceramic may for example comprise boron-nitride and/or silicon carbide.

Further, some polymers may exhibit a high thermal conductivity without fillers. For example, the carrier material 110 may comprise ultrahigh molecular weight polyethylene (UHMWPE), in particular UHMWPE-fibers. UHMWPE-fibers, in particular with a high crystallinity may exhibit a high thermal conductivity while being flexible.

In some embodiments, the carrier material 110 may comprise between about 0.1 wt.-% to about 70 wt.-%, more specifically between about 0.5 wt.-% to about 60 wt.-% and in particular between about 1.5 wt.-% to about 50 wt.-% of the inorganic filler, relative to the total weight of the carrier material 110 including the filler. An increased amount of filler may increase the thermal conductivity. A higher proportion of polymer may increase the flexibility and/or tear resistance of the carrier material 110.

The thermal conductivity of the carrier material 110 may be determined prior to applying the thermoresponsive material 120 thereto during production. Alternatively, the thermal conductivity of the carrier material 110 may be determined after removal of the thermoresponsive material 120 from the facial mask by physical, thermal or chemical methods.

In some embodiments, the facial mask 100 may comprise between about 10 wt.-% to about 80 wt.-%, more specifically between about 20 wt.-% to about 70 wt.-% and in particular between about 30 wt.-% to about 60 wt.-% of the carrier material 110, relative to the total weight of the facial mask 100 excluding water. In some embodiments, the facial mask 100 may comprise between about 10 wt.-% to about 80 wt.-%, more specifically between about 20 wt.-% to about 70 wt.-% and in particular between about 30 wt.-% to about 60 wt.-% of the thermoresponsive material 120, relative to the total weight of the facial mask 100 excluding water.

In some embodiments, the thermoresponsive material 120 may comprise a thermoresponsive polymer. The term "thermoresponsive polymer" is well known and i.a. (inter alia) attributed its common meaning in the art. Within this disclosure the term may in particular refer to polymers that exhibit a drastic and discontinuous change of their physical properties with temperature. Some thermoresponsive polymers may exhibit a drastic, discontinuous and reversible change of their conformation with temperature.

The thermoresponsive polymer may undergo a conformational change at a lower critical solution temperature, in particular the thermoresponsive polymer may be configured to be in an expanded state below the lower critical solution temperature and a collapsed state above the lower critical solution temperature. For example, the thermoresponsive polymer may undergo a coil-globule transition, wherein the thermoresponsive polymer collapses from an expanded coil state through an ideal coil state to a collapsed globule state. The thermoresponsive polymer may be more hydrophobic in its collapsed state compared to its expanded state. Without wishing to be bound by theory, in the expanded state the thermoresponsive polymer may form hydrogen bonds with surrounding water molecules leading to absorption. The hydrogen bonds may be formed in particular due to interactions with polar side groups of the thermoresponsive polymer. When the thermoresponsive polymer collapses, the water molecules may no longer be able to interact with the polar side groups as the thermoresponsive polymer coils up. As a result, the water may be released or desorbed. The lower critical solution temperature of the thermoresponsive material 120 may be between about 30°C to about 45°C, more specifically between about 33°C to about 40°C, and in particular between about 35°C to about 38°C. Hence, the threshold temperature may be the lower critical solution temperature. The lower critical solution temperature of the thermoresponsive material 120 may correspond to the lower critical solution temperature of the thermoresponsive polymer comprised therein. However, by adding for example a salt to the thermoresponsive material 120, the lower critical solution temperature may be adjusted.

In some embodiments, the thermoresponsive polymer may comprise poly(N-isopropylacrylamide) and/or poly(N-vinylcaprolactam).

In some embodiments, the thermoresponsive material 120 may comprise a hydrogel. The hydrogel may comprise a network of crosslinked polymer chains, wherein the crosslinked polymer chains comprise hydrophilic polymer chains. The hydrogel may increase the specific water absorption capacity of the thermoresponsive material 120. The term "specific water absorption capacity" within this disclosure may refer to the maximum weight of water absorbed per weight unit of an absorbent. While the thermoresponsive polymer may absorb water itself, mixing the thermoresponsive polymer with a hydrogel may lead to a higher specific water absorption capacity.

In some embodiments, the thermoresponsive material 120 may comprise an interpenetrating polymer network, more specifically an interpenetrating polymer network comprising the thermoresponsive polymer and the hydrogel.

The thermoresponsive polymer together with the hydrogel may form an interpenetrating polymer network. The term "interpenetrating polymer network" is well-known and i.a. attributed its common meaning in the art. Within this disclosure it may refer in particular to polymers comprising at least two different types of macromolecules. The macromolecules may form networks, wherein the networks may be at least partially interlaced on a polymer scale but not covalently bonded to each other. In embodiments, one of the at least two different types of macromolecules may be that constituting the thermoresponsive polymer and the other may be that constituting the hydrogel. As a result, the thermoresponsive polymer's macromolecules may interpenetrate the hydrogel's macromolecules. Without wishing to be bound by theory, it is believed that at temperatures below the lower critical solution temperature the interpenetrating polymer network may absorb water, in particular due to the presence of polar side groups. The absorption of water may lead to an increase in volume of the interpenetrating polymer network. At temperatures above the lower critical solution temperature the thermoresponsive polymer's macromolecules may collapse and release absorbed water. The collapse of the thermoresponsive polymer's macromolecules and/or the release of water may lead to a volume decrease of the interpenetrating polymer network which in turn may lead to a release of water by the hydrogel's macromolecules.

The synthesis of interpenetrating networks is known in the art. For example, in a first step, a polymer such as poly(N-isopropylacrylamide) may form a first three-dimensional network when cross-linked with a polymerizable monomer such as N,N'-methylene-bis-acrylamide. The first three-dimensional network may then be suspended in an aqueous solution comprising for example sodium alginate. The sodium alginate may then be crosslinked by the addition of CaCl₂ to form a second three-dimensional network which interlaces the first three-dimensional network.

In some embodiments, the hydrogel may comprise sodium alginate, agarose, methylcellulose, hyaluronan, collagen, gelatin, fibrin, elastin like polypeptides, polyvinyl alcohol, polyethylene glycol, sodium polyacrylate, acrylate polymers, copolymers thereof and/or mixtures thereof. In some embodiments, the hydrogel may comprise a polyacrylamide-carbon nanotube-calcium chloride hydrogel.

In some embodiments, the specific water absorption capacity of the thermoresponsive material 120 may be at least about 0.1 g/g, more specifically at least about 1.2 g/g, and in particular at least about 9.0 g/g. A thermoresponsive material 120 with a higher specific water absorption capacity may be able to absorb more water and as a result more thermal energy compared to a thermoresponsive material 120 with a lower specific water absorption capacity.

The specific absorption capacity may be measured by first immersing a sample of 1 g of the thermoresponsive material 120 in water for 24 hours. The used water shall be deionized water, class 3 according to OENORM EN ISO 3696:1995-08-01. Subsequently, the sample is freeze dried to remove the water. The freeze drying may be performed with any commercially available freeze dryer, as long as it can lower the water content below at least 4 wt.-%. Finally, the sample is weighed again, and the weight compared to the weight measured after immersion in water.

In some embodiments, the average molar mass of the thermoresponsive polymer may be between 1000 g/mol to about 100000 g/mol, more specifically between about 5000 g/mol to about 70000 g/mol and in particular between about 10000 g/mol to about 50000 g/mol. The average molar mass may be the weight-average molecular weight of the thermoresponsive polymer. The average molar mass may be measured according to ASTM D4001-20. A higher average molar mass of the thermoresponsive polymer may increase the lower critical solution temperature of the thermoresponsive material 120 and vice versa.

In some embodiments, the thermoresponsive material 120 may comprise between about 10 wt.-% to about 80 wt.-%, more specifically between about 20 wt.-% to about 70 wt.-% and in particular between about 30 wt.-% to about 60 wt.-% of the thermoresponsive polymer, relative to the total weight of the thermoresponsive material 120 excluding water.

In some embodiments, the thermoresponsive material 120 may comprise a salt, in particular potassium chloride. A higher amount of salt may increase the lower critical solution temperature of the thermoresponsive material 120 and vice versa.

In some embodiments, the thermoresponsive material 120 may further comprise one or more cross-linking agent(s) and/or reaction products of one or more cross-linking agent(s) and the thermoresponsive polymer, in particular di(ethylene glycol) divinyl ether and reaction products thereof. In some embodiments, the cross-linking agent may be N,N'-methylene-bis-acrylamide. The thermoresponsive polymer may form a three-dimensional network with the cross-linking agent(s). Cross-linking may be advantageous for forming and interpenetrating polymer network and/or for the mechanical stability of the thermoresponsive material 120.

In some embodiments, the thermoresponsive polymer and/or thermoresponsive material 120 may be more hydrophilic at temperatures below the lower critical solution temperature than at temperatures above the lower critical solution temperature. Accordingly, the thermoresponsive polymer and/or thermoresponsive material 120 may be more hydrophobic at temperatures above the lower critical solution temperature than at temperatures below the lower critical solution temperature. A change in hydrophilicity and hydrophobicity of the thermoresponsive polymer may allow the thermoresponsive polymer and/or thermoresponsive material 120 to absorb more water and an increased hydrophilicity may lead the thermoresponsive polymer and/or thermoresponsive material 120 to release more water.

In some embodiments, the facial mask 100 may comprise a bactericidal agent, in particular the thermoresponsive material 120 may comprise the bactericidal agent. The bactericidal agent may be configured to prevent fouling of the facial mask 100 between uses. Additionally or alternatively, the bactericidal agent may be configured to kill or inactivate bacteria present on the user's skin during application to reduce the risk of skin infections after shaving. The bactericidal agent may comprise for example colloidal silver, iodine or cationic surfactants, e.g. quaternary ammonium cations.

In some embodiments, the facial mask 100 may comprise skin care ingredients, in particular water-soluble skin care ingredients. For example, the facial mask 100 may comprise a cooling agent. The cooling agent may be configured to trigger cold-sensitive receptors, in particular the cold-sensitive TRPM-8 receptors, which may provide the user with a pleasant cooling sensation. For example, the cooling agent may be an essential oil, in particular menthol. Alternatively, the cooling agent may comprise for example icilin.

Further, the facial mask 100 may comprise butter(s) and/or oil(s). The butter(s) and the oil(s) may act as refatting ingredients, which may moisturize irritated skin and may help the skin regenerate.

In some embodiments, the butter(s) is (are) of plant origin, in particular such as those described in Ullmann's Encyclopedia of Industrial Chemistry ("Fats and Fatty Oils", A. Thomas, Published Online: 15 JUN 2000, DOI: 10.1002/14356007.a10_173, point 13.2.2.2. Shea Butter, Borneo Tallow, and Related Fats (Vegetable Butters)) which are incorporated herein by reference.

In some embodiments, the butter(s) comprise butters selected form the following list: Shea butter, Nilotica shea butter (Butyrospermum parkii), galam butter (Butyrospermum parkii), Borneo butter or fat or tengkawang tallow (Shorea stenoptera), shorea butter, illipe butter, madhuca butter or (Bassia) Madhuca longifolia butter, mowrah butter (Madhuca latifolia), katiau butter (Madhuca mottleyana), phulwara butter (M. butyracea), mango butter (Mangifera indica), murumuru butter (Astrocaryum murumuru), kokum butter (Garcinia indica), ucuuba butter (Virola sebifera), tucuma butter, painya (kpangnan) butter (Pentadesma butyracea), coffee butter (Coffea arabica), apricot butter (Prunus armeniaca), macadamia butter (Macadamia ternifolia), grapeseed butter (Vitis vinifera), avocado butter (Persea gratissima), olive butter (Olea europaea), sweet almond butter (Prunus amygdalus dulcis), cocoa butter (Theobroma cacao) and sunflower butter.

Within the present disclosure, the term "oil" refers to C₈-C₅₀-substances that are liquid at room temperature (about 25°C). These oils may be hydrocarbon-based oils of animal, plant, mineral or synthetic origin, silicone oils and/or fluoro oils, and mixtures thereof. The term "hydrocarbon-based oil" refers to an oil mainly containing carbon atoms and hydrogen atoms (e.g. at least about 70 or about 80 atomic wt.-%).

Specific examples of oils that can be used include:
- hydrocarbon-based oils of animal origin, such as perhydrosqualene (squalane);
- hydrocarbon-based plant oils, such as liquid triglycerides of fatty acids of 4 to 10 carbon atoms, such as heptanoic or octanoic acid triglyceride; coconut oil, sunflower oil, corn oil, soybean oil, marrow oil, grapeseed oil, sesame oil, hazelnut oil, apricot oil, macadamia oil, arara oil, castor oil, avocado oil, caprylic/capric acid triglycerides, and jojoba oil;
- linear or branched hydrocarbons of mineral or synthetic origin, such as liquid paraffin and derivatives thereof, petroleum jelly, polydecenes and hydrogenated polyisobutene, such as parleam;
- synthetic esters and ethers, in particular, of fatty acids, such as the oils of formula R₁COOR₂ in which R₁ represents a fatty acid residue containing from about 7 to about 25 carbon atoms and R₂ represents a hydrocarbon-based chain containing from about 3 to about 25 carbon atoms, such as, for example, purcellin oil, isononyl isononanoate, isopropyl myristate, 2-ethylhexyl palmitate, 2-octyldodecyl stearate, 2-octyldodecyl erucate, isostearyl isostearate; hydroxylated esters such as isostearyl lactate, octyl hydroxystearate, octyldodecyl hydroxystearate, diisostearyl malate, triisocetyl citrate, fatty alkyl heptanoates, octanoates or decanoates; polyol esters such as propylene glycol dioctanoate, neopentyl glycol diheptanoate, diethylene glycol diisononanoate; and pentaerythritol esters such as pentaerythrityl tetraisostearate.

In some embodiments the one or more oils may comprise an oil selected chosen from triglycerides and/or triglyceride derivatives and in particular a coconut oil.

In some embodiments, it may be advantageous that the one or more oils comprises a glycerol esterified with three fatty acids. The fatty acids may be as defined above.

In a second aspect, the present disclosure relates to a method for producing a facial mask 100, wherein the method comprises embedding a material configured to release water above a threshold temperature and absorb water below a threshold temperature into a carrier material 110 exhibiting a thermal conductivity of at least 0.1 W/mK.

Although the present disclosure is defined in the attached claims, it should be understood that the present disclosure can also (alternatively) be defined in accordance with the following embodiments:
1. A facial mask (100) comprising a carrier material (110) and a thermoresponsive material (120) configured to release water above a threshold temperature and absorb water below a threshold temperature, wherein the facial mask (100) exhibits a thermal conductivity of at least 0.1 W/mK at 20°C, when the facial mask (100) is saturated with water.
2. The facial mask (100) according to embodiment 1, wherein the threshold temperature is between about 30°C to about 45°C, more specifically between about 33°C to about 40°C, and in particular between about 35°C to about 38°C.
3. The facial mask (100) according to any preceding embodiment, wherein the facial mask (100) exhibits a thermal conductivity of at least 0.2 W/mK, more specifically at least 1 W/mK and in particular at least 5 W/mK at 20°C, when the facial mask (100) is saturated with water.
4. The facial mask (100) according to any preceding embodiment, wherein the carrier material (110) exhibits a thermal conductivity of at least 0.2 W/mK, more specifically at least 1 W/mK and in particular at least 5 W/mK at 20°C.
5. The facial mask (100) according to any preceding embodiment, wherein the carrier material (110) is flexible.
6. The facial mask (100) according to any preceding embodiment, wherein the facial mask (100) comprises between about 10 wt.-% to about 80 wt.-%, more specifically between about 20 wt.-% to about 70 wt.-% and in particular between about 30 wt.-% to about 60 wt.-% of the carrier material (110), relative to the total weight of the facial mask (100) excluding water.
7. The facial mask (100) according to any preceding embodiment, wherein the facial mask (100) comprises between about 10 wt.-% to about 80 wt.-%, more specifically between about 20 wt.-% to about 70 wt.-% and in particular between about 30 wt.-% to about 60 wt.-% of the thermoresponsive material (120), relative to the total weight of the facial mask (100) excluding water.
8. The facial mask (100) according to any preceding embodiment, wherein the carrier material (110) exhibits a Young's modulus between about 0.1 GPa to about 60 GPa, more specifically 0.5 GPa to about 10 GPa and in particular, between about 1 GPa to about 5 GPa.
9. The facial mask (100) according to any preceding embodiment, wherein the carrier material (110) comprises a polymer.
10. The facial mask (100) according to any preceding embodiment, wherein the carrier material (110) comprises fibers, in particular polymer fibers.
11. The facial mask (100) according to any preceding embodiment, wherein the carrier material (110) is a woven material, in particular wherein the carrier material (110) is a woven fabric.
12. The facial mask (100) according to any preceding embodiment, wherein the thermoresponsive material (120) is embedded within the carrier material (110).
13. The facial mask (100) according to any one of embodiments 10 to 12, wherein the fibers have a diameter between about 100 µm to about 2000 µm, more specifically between about 300 µm to about 1000 µm.
14. The facial mask (100) according to any one of embodiments 9 to 13, wherein the carrier material (110) comprises fillers configured to increase the carrier material's (110) thermal conductivity, more specifically inorganic fillers and in particular a metal, graphite, carbon nanotubes and/or a ceramic.
15. The facial mask (100) according to embodiment 14, wherein the filler is in the form of particles, sheets or fibers, in particular sheets or fibers.
16. The facial mask (100) according to any one of embodiments 14 or 15, wherein the metals comprise copper particles and/or copper fibers.
17. The facial mask (100) according to any one of embodiments 14 to 16, wherein the ceramic comprises boron-nitride.
18. The facial mask (100) according to any one of embodiments 10 to 17, wherein the carrier material (110) comprises between about 0.1 wt.-% to about 70 wt.-%, more specifically between about 0.5 wt.-% to about 60 wt.-% and in particular between about 1.5 wt.-% to about 50 wt.-% of the inorganic filler, relative to the total weight of the carrier material (110) including the filler.
19. The facial mask (100) according to any preceding embodiment, wherein the carrier material (110) comprises Ultra High Molecular Weight Polyethylene.
20. The facial mask (100) according to any preceding embodiment, wherein the thermoresponsive material (120) comprises a thermoresponsive polymer.
21. The facial mask (100) according to any preceding embodiment, wherein the thermoresponsive material (120) comprises a hydrogel.
22. The facial mask (100) according to any preceding embodiment, wherein the thermoresponsive material (120) comprises an interpenetrating polymer network, more specifically an interpenetrating polymer network comprising the thermoresponsive polymer and the hydrogel.
23. The facial mask (100) according to any one of embodiments 20 to 22, wherein the thermoresponsive polymer undergoes a conformational change at a lower critical solution temperature, in particular wherein the thermoresponsive polymer is configured to be in an expanded state below the lower critical solution temperature and a collapsed state above the lower critical solution temperature.
24. The facial mask (100) according to any preceding embodiment, wherein the lower critical solution temperature of the thermoresponsive material (120) is between about 30°C to about 45°C, more specifically between about 33°C to about 40°C, and in particular between about 35°C to about 38°C.
25. The facial mask (100) according to embodiment 23 or 24, wherein the threshold temperature is the lower critical solution temperature.
26. The facial mask (100) according to any one of embodiments 20 to 25, wherein the thermoresponsive polymer comprises poly(N-isopropylacrylamide) and/or poly(N-vinylcaprolactam).
27. The facial mask (100) according to any one of embodiments 21 to 26, wherein the hydrogel comprises a network of crosslinked polymer chains, wherein the crosslinked polymer chains comprise hydrophilic polymer chains.
28. The facial mask (100) according to any one of embodiments 21 to 27, wherein the hydrogel comprises sodium alginate, agarose, methylcellulose, hyaluronan, collagen, gelatin, fibrin, elastin like polypeptides, polyvinyl alcohol, polyethylene glycol, sodium polyacrylate, acrylate polymers, copolymers thereof and/or mixtures thereof.
29. The facial mask (100) according to any one of embodiments 21 to 28, wherein the hydrogel comprises a polyacrylamide-carbon nanotube-calcium chloride hydrogel.
30. The facial mask (100) according to any preceding embodiment, wherein the specific water absorption capacity of the thermoresponsive material (120) is at least about 0.1 g/g, more specifically at least about 1.2 g/g, and in particular at least about 9.0 g/g.
31. The facial mask (100) according to any one of embodiments 20 to 30, wherein the average molar mass of the thermoresponsive polymer is between 1000 g/mol to about 100000 g/mol, more specifically between about 5000 g/mol to about 70000 g/mol and in particular between about 10000 g/mol to about 50000 g/mol.
32. The facial mask (100) according to any one of embodiments 20 to 31, wherein the thermoresponsive material (120) comprises between about 10 wt.-% to about 80 wt.-%, more specifically between about 20 wt.-% to about 70 wt.-% and in particular between about 30 wt.-% to about 60 wt.-% of the thermoresponsive polymer, relative to the total weight of the thermoresponsive material (120) excluding water.
33. The facial mask (100) according to any preceding embodiment, wherein the thermoresponsive material (120) further comprises a salt, in particular potassium chloride.
34. The facial mask (100) according to any one of embodiments 20 to 33, wherein the thermoresponsive polymer is more hydrophilic at temperatures below the lower critical solution temperature than at temperatures above the lower critical solution temperature.
35. The facial mask (100) according to any one of embodiments 20 to 34, wherein the thermoresponsive polymer is more hydrophobic at temperatures above the lower critical solution temperature than at temperatures below the lower critical solution temperature.
36. The facial mask (100) according to any preceding embodiment, wherein the facial mask (100) comprises a bactericidal agent, in particular wherein the thermoresponsive material (120) comprises the bactericidal agent.
37. The facial mask (100) according to any preceding embodiment, wherein the facial mask (100) comprises skin care ingredients, in particular water-soluble skin care ingredients.
38. The facial mask (100) according to any preceding embodiment, wherein the facial mask (100) comprises a cooling agent.
39. The facial mask (100) according to embodiment 38, wherein the cooling agent is configured to trigger cold-sensitive receptors, in particular the cold-sensitive TRPM-8 receptors.
40. The facial mask (100) according to embodiment 38 or 39, wherein the cooling agent is an essential oil, in particular menthol.
41. Method for producing a facial mask (100), wherein the method comprises embedding a material configured to release water above a threshold temperature and absorb water below a threshold temperature into a carrier material (110) exhibiting a thermal conductivity of at least 0.1 W/mK.

## Claims

1. A facial mask (100) comprising a carrier material (110) and a thermoresponsive material (120) configured to release water above a threshold temperature and absorb water below a threshold temperature, wherein the facial mask (100) exhibits a thermal conductivity of at least 0.1 W/mK at 20°C, when the facial mask (100) is saturated with water.

2. The facial mask (100) according to claim 1, wherein the threshold temperature is between about 30°C to about 45°C, more specifically between about 33°C to about 40°C, and in particular between about 35°C to about 38°C.

3. The facial mask (100) according to any preceding claim, wherein the facial mask (100) exhibits a thermal conductivity of at least 0.2 W/mK, more specifically at least 1 W/mK and in particular at least 5 W/mK at 20°C, when the facial mask (100) is saturated with water.

4. The facial mask (100) according to any preceding claim, wherein the facial mask (100) comprises between about 10 wt.-% to about 80 wt.-%, more specifically between about 20 wt.-% to about 70 wt.-% and in particular between about 30 wt.-% to about 60 wt.-% of the carrier material (110), relative to the total weight of the facial mask (100) excluding water.

5. The facial mask (100) according to any preceding claim, wherein the facial mask (100) comprises between about 10 wt.-% to about 80 wt.-%, more specifically between about 20 wt.-% to about 70 wt.-% and in particular between about 30 wt.-% to about 60 wt.-% of the thermoresponsive material (120), relative to the total weight of the facial mask (100) excluding water.

6. The facial mask (100) according to any preceding claim, wherein the carrier material (110) is flexible, more specifically wherein the carrier material (110) exhibits a Young's modulus between about 0.1 GPa to about 60 GPa, more specifically 0.5 GPa to about 10 GPa and in particular, between about 1 GPa to about 5 GPa.

7. The facial mask (100) according to any preceding claim, wherein the carrier material (110) comprises a polymer and/or wherein the carrier material (110) comprises fibers, more specifically polymer fibers.

8. The facial mask (100) according to any preceding claim, wherein the carrier material (110) is a woven material, in particular wherein the carrier material (110) is a woven fabric.

9. The facial mask (100) according to any preceding claim, wherein the thermoresponsive material (120) is embedded within the carrier material (110).

10. The facial mask (100) according to any one of claims 7 to 9, wherein the fibers have a diameter between about 100 µm to about 2000 µm, more specifically between about 300 µm to about 1000 µm.

11. The facial mask (100) according to any preceding claim, wherein the carrier material (110) comprises Ultra High Molecular Weight Polyethylene.

12. The facial mask (100) according to any preceding claim, wherein the thermoresponsive material (120) comprises a thermoresponsive polymer, in particular wherein the thermoresponsive polymer comprises poly(N-isopropylacrylamide) and/or poly(N-vinyl caprolactam).

13. The facial mask (100) according to any preceding claim, wherein the thermoresponsive material (120) comprises an interpenetrating polymer network, more specifically an interpenetrating polymer network comprising the thermoresponsive polymer and a hydrogel.

14. The facial mask (100) according to any preceding claim, wherein the facial mask (100) comprises a bactericidal agent, in particular wherein the thermoresponsive material (120) comprises the bactericidal agent and/or wherein the facial mask (100) comprises skin care ingredients, in particular water-soluble skin care ingredients.

15. Method for producing a facial mask (100), wherein the method comprises embedding a material configured to release water above a threshold temperature and absorb water below a threshold temperature into a carrier material (110) exhibiting a thermal conductivity of at least 0.1 W/mK.
